Europäisches Patentamt

European Patent Office

Office européen des brevets

(11) Numéro de publication: **0 241 963**

**A1**

## (12) DEMANDE DE BREVET EUROPEEN

(21) Numéro de dépôt: **87200474.2**

(22) Date de dépôt: **13.03.87**

(51) Int. Cl.⁴: **C12N 15/00** , A01H 1/00

(30) Priorité: **26.03.86 LU 86372**

(43) Date de publication de la demande:
**21.10.87 Bulletin 87/43**

(84) Etats contractants désignés:
**AT BE CH DE ES FR GB GR IT LI LU NL SE**

(71) Demandeur: **"Centre d'Etude de l'Energie Nucléaire", "C.E.N."**
**Rue Charles Lemaire 1**
**B-1160 Bruxelles(BE)**

(72) Inventeur: **Ledoux, Lucien Gaspard Henri**
**Donk 2**
**B-2360 Oud-Turnhout(BE)**

(74) Mandataire: **Debrabandere, René et al**
**Vereenigde Octrooibureaux Belgie N.V.**
**Charlottalei 48**
**B-2018 Antwerpen(BE)**

(54) **Procédé de traitement de matériel végétal afin d'obtenir l'expression d'au moins un gène, et matériel végétal dans lequel ce gène s'exprime.**

(57) On met à incuber après stérilisation, au moins une graine sèche de plante supérieure, au moins une spore sèche de plante inférieure ou au moins un embryon de plante excisé sec, dont la teneur en humidité est inférieure à 10 % en poids, au moins jusqu'à ce que l'embryon ait triplé de taille, dans une solution aqueuse d'ADN comprenant le gène étranger complet et/ou le cADN de ce gène.

EP 0 241 963 A1

**"Procédé de traitement de matériel végétal afin d'obtenir l'expression d'au moins un gène,et matériel végétal dans lequel ce gène s'exprime".**

L'invention concerne un procédé de traitement de matériel végétal afin d'obtenir l'expression d'au moins un gène dans des cellules végétales, selon lequel on stérilise d'abord le matériel végétal et on le met ensuite à incuber dans une solution d'ADN possédant au moins un gène étranger à exprimer et/ou le cADN de ce gène.

Il est déjà connu que l'introduction dans des cellules de plantes de gènes étrangers peut être réalisée par absorption directe d'ADN par des cellules végétales.

En particulier, la transformation de protoplastes de plantes a déjà été réalisée par simple incubation avec un plasmide possédant le gène étranger.

Ainsi en travaillant sur des protoplastes isolés, il a déjà été possible de transformer des monocotylées tels que la céréale TRITICIUM MONOCOCCUM (Mol.Gen.Genet.199, p. 178 par LÖRZ H., BAKER B. et SCHELL J. - 1985) ou la graminée LOLIUM MULTIFLORUM (Mol.Gen.Genet 199, p. 183 par POTRYKUS I., SAUL M.W., PETRUSKA J.,PASZKOWSKI J. et SHILLITO D.-1985). Dans ces deux cas, comme souvent pour les protoplastes transformés, il n'a pas encore été possible de régénérer des plantes.

Il semble plus facile de pouvoir traiter le matériel végétal entier tel que la graine ou l'embryon excisé et des recherches ont été faites dans ce sens.

On savait déjà que, bien que la graine soit un système complexe par, notamment, la présence de parois cellulaires et l'exsudation possible, chez certaines espèces, de nucléases dans le milieu d'incubation, de l'ADN exogène pouvait pénétrer dans les cellules de graine et dans l'embryon (J. Mol. Biol. 43, 243 - LEDOUX L. et HUART L.; 1969) et que seul un faible pourcentage de l'ADN était dégradé lors de la prise si la variété de graine était bien choisie (Europ. J. Biochem. 23, 96 LEDOUX L. et al; 1971).

Des mutants thiamine d'ARABIDOPSIS THALIANA ont déjà été corrigés par un traitement avec des ADN bactériens adéquats (Nature 249 , 17 LEDOUX L. et al; 1974 et Canad. J. Genet.Cyt.21, 515; LEDOUX L. et al;1979).

On en avait déduit que l'ADN bactérien incubé avec des graines pouvait être, après la prise, exprimé par les cellules de la plante.

Un procédé de traitement de graines dans une solution d'ADN plasmidien a été décrit dans Arch. Int. Physiol. Biochem. 90, B 44 (LEDOUX L. et al -1982). Cet article décrit le transfert direct et l'expression d'ADN plasmiden dérivé du plasmide pBR 325 portant le gène de résistance à l'ampicilline codant pour la β-lactamase.

Les procédés pour traiter le matériel végétal tels que décrits dans les publications mentionnées ci-devant ne permettent cependant que dans de rares cas à l'homme de métier d'obtenir de bons résultats. En partculier l'absorption de l'ADN par les monocotylées s'avère dans la plupart des cas difficile à réaliser.

L'invention a pour but de procurer un procédé simple mais applicable en général et permettant donc d'obtenir l'expression d'un gène par un nombre élevé de variétés de plantes.

Dans ce but, on met à incuber au moins une graine sèche de plante supérieure, au moins une spore sèche de plante inférieure ou au moins un embryon de plante excisé sec dont la teneur en humidité est inférieure à 10 % en poids, au moins jusqu'à ce que l'embryon ait triplé de taille, dans une solution d'ADN comprenant le gène étranger complet et/ou le cADN de ce gène.

On a constaté qu'en appliquant ce procédé de l'ADN est absorbé aussi bien par des monocotylées que par des dicotylées.

De préférence, on met à incuber au moins une graine, une spore ou un embryon dont la teneur en humidité est inférieure à 5 % en poids.

Bien que sous les conditions susdites l'activité nucléasique est peu importante, il peut être utile de sélectionner d'une plante déterminée les variétés les plus pauvres en nucléases.

La stérilisation nécessaire avant l'incubation peut avoir pour conséquence que la teneur en humidité du matériel végétal devient trop élevée. Dans ce cas, ou dans le cas où le matériel végétal à traiter possède déjà de sa nature une teneur en humidité trop élevée, on sèche le matériel végétal avant qu'on le fait incuber dans la solution d'ADN.

Dans une forme de réalisation particulière de l'invention on fait incuber des graines pelables et on les pèle avant de les incuber dans la solution d'ADN.

Dans une forme de réalisation remarquable de l'invention, on utilise comme solution d'ADN une solution d'ADN comprenant au moins un gène étranger eucaryote et/ou le cADN de ce gène.

Ce gène eucaryote est par exemple le gène codant pour l'hormone de croissance humaine (hGH).

L'ADN peut contenir le gène étranger tel quel (éventuellement avec introns et promoteur) par exemple un gène synthétique ou manipulé ou le cADN tel quel de ce gène.

Il peut aussi être un vecteur d'expression, par exemple un plasmide, un cosmide ou un phage comprenant le gène étranger et/ou le cADN de ce gène.

L'invention concerne également le matériel végétal obtenu par le procédé susdit et dans lequel le gène s'exprime.

L'invention concerne ainsi entre autres une graine, une spore ou un embryon de plante excisé dans laquelle ou lequel s'exprime un gène étranger, cette graine ou spore ou cet embryon ayant absorbé de l'ADN comprenant le gène étranger à exprimer ou le cADN de ce gène selon le procédé susdit.

En particulier, le matériel végétal a absorbé de l'ADN comprenant le gène complet codant pour l'hormone de croissance humaine ou le cADN de ce gène.

L'invention concerne également les plantes, les cals ou les cellules obtenus à aprtir d'une graine, d'une spore ou d'un embryon traité par le procédé selon l'invention susdit dans lesquels ou lesquelles le gène étranger s'exprime ainsi que les descendants y compris les hybrides, de cette plante.

D'autres particularités et avantages de l'invention, ressortiront de la description d'un procédé de traitement de matériel végétal afin d'obtenir l'expression d'au moins un gène, et du matériel végétal dans lequel ce gène s'exprime, selon l'invention; cette description n'est donnée ci-après qu'à titre d'exemple non limitatif et avec référence aux dessins annexés.

La figure 1 représente la structure d'un vecteur d'expression pouvant être utilisée dans le procédé selon l'invention.

La figure 2 représente la structure d'un autre vecteur d'expression pouvant être utilisé dans le procédé selon l'invention.

Selon l'invention on fait germer le matériel végétal stérile sec dans une solution d'ADN comprenant le gène étranger qu'on veut faire exprimer par les cellules végétales.

Le matériel végétal susdit est constitué par une graine, une spore ou un embryon excisé.

La graine ou l'embryon peut être aussi bien d'un monocotylée, par exemple le riz ou la fléole des prés, que d'un dicotylée comme l'Arabidopsis Thaliana.

On commence par stériliser le matériel végétal par un moyen chimique adéquat, avec ou sans adjuvant tel qu'un tensioactif.

Ensuite on met le matériel végétal en germination dans la solution d'ADN.

L'incubation doit se faire au moins jusqu'au début visible de la plantule ou plus particulièrement au moins jusqu'à ce que l'embryon ait triplé de taille.

Ce triplage de taille arrive dans la plupart des cas après les premières 72 heures et dans certaines cas déjà après les premières 48 heures du développement de la plantule à partir du germe.

On a constaté qu'à ce stade de la germination, stade qui est très particulier pour le métabolisme, il ne se forme pas de nucléases et les cellules acceptent entre autres de l'ADN avec le gène étranger, à condition toutefois que ces cellules soient suffisamment sèches. Lorsque les cellules se sont hydratées, elles se ferment pour l'ADN étranger.

La teneur maximale d'eau admissible varie avec la variété de plante mais est en tout cas inférieure à 10 % en poids et de préférence inférieure à 5 % en poids.

Si nécessaire on commence par sécher le matériel végétal, soit à l'air libre, soit par séchage chimique soit encore par lyophilisation.

Un tel séchage peut s'avérer nécessaire lorsque le matériel végétal contient trop d'humidité de sa nature mais surtout si le matériel végétal a absorbé de l'humidité lors de la stérilisation par voie humide.

Si le matériel végétal est une graine, on la pèle en enlevant glumes et glumelles, avant l'incubation chaque fois qu'on le peut. Ceci favorise l'absorption de l'ADN. Un tel pelage est par exemple indiqué pour la plupart des céréales comme l'orge. Pour favoriser ce pelage, on mouille d'abord la graine pendant 30 minutes dans de l'eau. Ceci a normalement pour conséquence qu'il est nécessaire de sécher ou de resécher la graine avant de l'incuber dans la solution d'ADN.

La solution aqueuse d'ADN peut être une solution saline ou une solution tamponnée.

L'ADN peut être aussi bien sous forme linéaire que sous forme circulaire ou superhélicoïdale. Il contient un ou plusieurs gènes étrangers complets (éventuellement avec promoteur et introns) et/ou le cADN de ce gène.

Ce gène peut aussi être un gène manipulé pour favoriser son expression, par exemple selon une technique connue par laquelle il est placé en aval de promoteurs particuliers ou de séquences UAS (Upstream Activating Sequences) et en amont de séquences de polyadenylation ou de terminaison.

L'ADN peut être le gène étranger, manipulé ou non, tel quel ou le cADN tel quel de ce gène.

L'ADN peut être également constitué d'un vecteur d'expression comprenant ce gène et/ou le cADN de ce gène.

Le gène étranger ou le cADN de ce gène peut ainsi être inséré dans au moins un plasmide en particulier un plasmide ou vecteur de départ de la série pBR (pBR 322,325, 328 etc.),au moins un cosmide, au moins un phage ou une combinaison de ces vecteurs.

On utilise une solution d'ADN ayant une concentration d'au moins 1 µg/ml. La concentration minimale dépend du matériel végétal, mais dans la plupart des cas on préfère une concentration voisine de 400 à 500 mg/ml.

Afin de permettre, après l'application du procédé, une sélection du matériel végétal transformé par le procédé on peut incuber le matériel végétal à traiter dans une solution d'ADN ne comprenant pas seulement ces gènes codant pour une protéine désirée et/ou le cADN de ce gène mais également un gène codant pour la résistance à une substance toxique par exemple à une antibiotique, à des métaux lourds , aux organochlorés ou aux herbicides.

La sélection du matériel végétal transformé peut avantageusement être effectuée sur la descendance des plantes obtenues à partir du matériel végétal traité.

Le gène étranger en question peut être aussi bien eucaryotique que procaryotique ou viral.

Un gène eucaryotique intéressant à faire exprimer est le gène de l'hormone de croissance humaine (hGH).

Bien que, lorsque le traitement par l'ADN se fait tout au début de la germination, les cellules forment peu de nucléases, on a constaté que pour une plante donnée certaines variétés de cette plante produisent moins de nucléases que d'autres. Il est dès lors utile de sélectionner avant tout par dosage biochimique, les variétés d'une plante les plus pauvres en nucléases.

Après l'incubation du matériel végétal dans la solution d'ADN, on met le matériel traité dans un milieu de culture approprié permettant le développement de plantes ou de cals formés à partir du matériel végétal traité ou de parties, par exemple des cellules isolées de ce matériel.

On cultive les plantes ou les cals jusqu'à la récolte de graines ou de spores qu'on cultive à leur tour. On traite alors les descendants des plantes pour en extraire par des méthodes connues la protéine exprimée par le gène étranger absorbé par le matériel végétal traité à l'origine.

Les exemples données ci-après illustrent l'invention plus en détail.

## Exemple 1

### 1. Préparation des graines:

On agite des graines d'orge (HORDEUM VULGARE L. var. Trumph) pendant 15 min dans une solution de 10 µl de polyoxyéthylène sorbitan monolaureate (Tween 20R) pour 20 ml d'eau.
On pèle ensuite soigneusement les graines.
On les stérilise pendant 60 min par de l'hypochlorite de sodium (13 % de chlore actif) additionnée de 10 µl de Tween 20 par 20 ml.

Après lavage à l'eau stérile jusqu'à un pH de 6, on sèche les graines en milieu stérile à température ambiante jusqu'à ce que la teneur en humidité soit inférieure à 5 % en poids.

Quand les graines sont fraîches et que l'embryon apparaît comme très hydraté , on peut sécher les graines pendant une nuit sur drierite ou on peut les lyophiliser pendant une nuit avant de les stériliser.

### 2. Préparation de la solution d'ADN.

On prépare une culture bactérienne à partir de la souche E.Coli K 12-HB 101 contenant le plasmide ou vecteur d'expression c hGH 800/pBR 322 (appelé ci-après pc hGH 800) dans un milieu complet de Luria (milieu L), auquel on a ajouté, après ajustement du pH à 7,5 avec du NaOH 1 N et stérilisation, 50 mg/l d'ampicilline filtrée sur millipore.

La préparation du plasmide susdit est décrite en détail dans Science 205, 602-607 (1979) par J.A. MARTIAL, R.A. HALLEWELL, J.D. BAXTER et H.M. GOODMAN.

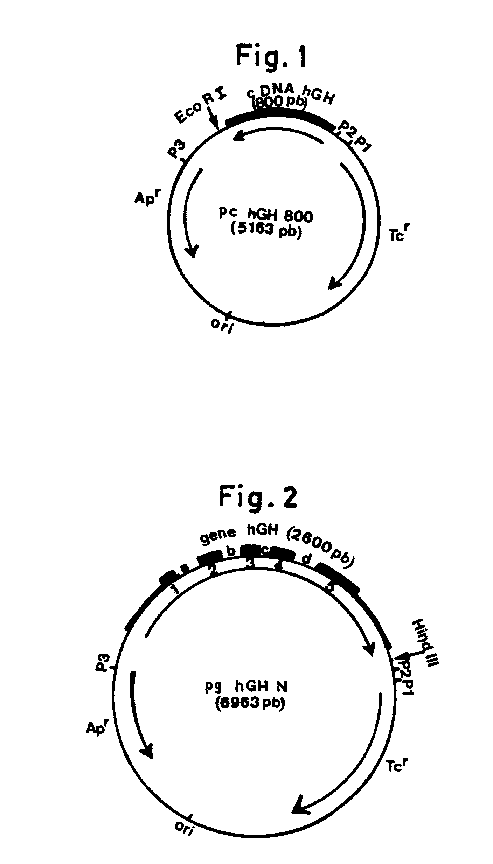

Ce vecteur d'expression a été préparé en insérant le fragment d'ADN de 800 pb (paires de bases) comprenant les 800 pb du cADN du gène hGH c.-à-d. le gène de l'hormone de croissance humaine, au site Hind III du pBR 322 inséré dans la souche E. coli CM 240. Ce fragment d'ADN a été placé dans le même sens de lecture que le gène codant pour la résistance à l'ampicilline. La structure du vecteur plasmidien d'expression pc hGH 800 est représentée à la figure 1 dans laquelle
P1 est le promoteur principal du gène bla (Apʳ) dans le pBR 322,
P3 est le promoteur naturel de ce gène (P1 et P2 font partie de la zone "promoteur" du gène Tc ʹ), et
ori est l'origine de réplication.

On prépare la culture de la manière suivante. On prépare d'abord une préculture avec 40 ml du milieu L et 50 mg/l d'Ap. On laisse le mélange sous agitation à 37°C pendant la nuit. Le lendemain, on ensemence 1 litre de ce même milieu avec la souche. On laisse les bactéries se multiplier jusqu'à ce que $A_{660}$ atteingne 0,4 à 0,6. On ajoute alors du chloramphénicol à raison de 250 mg/l. Cet antibiotique a pour effet de bloquer la synthèse des protéines et d'empêcher la réplication de l'ADN chromosomique. L'ADN plasmidien continue, par contre, à se répliquer. On amplifie les plasmides pendant 12 à 16 heures.

Ensuite on effectue la lyse de cellules des bactéries par la méthode de lyse alcaline à grande échelle décrite par MANIATIS et al., in "Molecular Closing "Cold-Spring Harbor Laboratory (1982).

On isole ensuite et on purifie l'ADN plasmidien en gradient de CsCl en présence de bromure d'éthidium.

A chaque ml de la solution, on ajoute 1,0 gr de chlorure de césium. On additionne de 4,5 ml de bromure d'éthidum à 10 mg/ml. On ajuste la densité finale de la solution au réfractomètre : l'indice de réfraction doit être égal à 1,386. Il se forme des aggrégats qu'on enlève par centrifugation.

On effectue une première centrifugation pendant 48 heures à 36.000 tpm à 25°C (MARTIN CHRIST Omega II, rotor 65 Ti.).

L'ADN plasmidien est isolé à l'aide d'une seringue et une deuxième ultracentrifugation est réalisée à 45 000 tpm pendant 48 heures . La bande d'ADN plasmidien est isolée. On y ajoute 2 volumes d'isobutanol saturé en eau et on agite plusieurs fois pour éliminer le bromure d'éthidum soluble dans l'isobutanol. Après centrifugation l'isobutanol est enlevé. On répète 4 fois cette opération. L'élimination du CsCl est réalisée par dialyse pendant plusieurs heures contre une solution de 0,01 M Na Cl renouvelée 2 à 3 fois.

On précipite l'ADN en y ajoutant 1/10 de volume de NaCl 5M et deux volumes d'éthanol. On laisse à -20°C pendant 1 h et on centrifuge 10 minutes à 10 000 tpm (Sorvall RC-5,4°C). On élimine l'alcool et on dissout le culot dans un volume de NaCl 0,01 M stérile de manière à obtenir une concentration finale d'ADN voisine de 400 µg/ml. On garde le plasmide c hGH 800 en présence de chloroforme à 4°C.

### 3. Traitement des graines dans la solution d'ADN.

On place les graines individuellement dans les alvéoles d'une plaque de porcelaine dans 150 µl de solution de plasmide.

On humidifie les papiers absorbants sur lesquels repose la plaque de porcelaine pour éviter une évaporation des solutions. On scelle alors la boîte de Pétri à l'aide de papier isolant.

On enveloppe la boîte de Pétri dans du papier aluminium pour éviter une germination trop rapide. On maintient la boîte de Petri à 20°C pendant 3 jours. Après deux jours, on effectue un contrôle de stérilité en posant une anse de platine entre les graines et en plaçant une partie des graines dans le milieu de Luria contenant par litre :
-10 gr. de bactotryptone
-5 gr. de bacto-yeast extract
-5 gr. de NaCl
-1 gr. de glucose

La solidification du milieu a été réalisée par l'addition de 15 g/l d'agar.

On strie alors une boîte de Pétri contenant un milieu gélosé riche et on la place à 27°C. Les conditions permettent le développement de la plupart des moisissures et des bactéries pouvant coloniser les graines. On garde la boîte en observation pendant plusieurs jours.

### 4. Culture de plantes.

Lorsque l'incubation est terminée, on reprend les graines et on les dépose dans un tube contenant 6 ml de milieu de culture gélosé.

Ce milieu de culture est le milieu "Brown" (décrit par Miksche J.P. et Brown J.A.M. dans Am.J.Bot 52, 533 (1965)) à pH 5,8 permettant le développement de plantes à partir des graines.

On solidifie le milieu avec 7,8 g/l d'ager (oxoïd) qu'on dissout par chauffage pendant 3 minutes à 110°C. On répartit le milieu dans des tubes à raison de 6 ml dans chaque tube et on stérilise (15 minutes à 110°C).

On contrôle les conditions de culture à trois niveaux,c'est-à-dire au niveau de :
-la température (22°C)
-l'intensité lumineuse (10.000 lux et 16 heures par jour)
-l'humidité (65%)

### 5. Extraction de l'hormone de croissance.

On place les tissues des plantes dans un tube "micro-Potter" où elles sont broyées en présence de 200 µl de tampon phosphate glacé 0,1 M pH 6,8, Triton X-100 0,25 %. Le tube dans lequel se fait l'homogénéisation est plongé dans un autre tube contenant de la glace. Le broyage se fait à l'aide d'un homogénéisateur (TRI-R STIR-R,S63C à 1200 tpm). On réalise 50 passages puis on centrifuge pendant 2 minutes à 9000 g (BECKMAN Microfuge B) ce qui permet d'éliminer une partie des débris cellulaires. Le surnageant est récupéré et déposé dans un tube eppendorf mis dans de la glace. Le culot est repris dans 200 $\mu$l de tampon phosphate-triton et resoumis à 50 passages. Cette opération est répétée jusqu'à ce que le culot soit blanc. On collecte le surnageant.

On précipite l'hormone de croissance humaine entre 25 % et 60 % de saturation en sulfate d'ammonium. On solubilise le précipité dans un tampon phosphate et on isole l'hormone par chromatographie sur SEPHADEX G 75. On purifie ensuite l'hormone isolée selon des techniques connues.

## Exemple 2.

On répète l'exemple 1 mais au lieu de placer les graines traitées à l'ADN dans un milieu de culture Brown on les met dans des boîtes de Pétri contenant un milieu B5 induisant la formation de cals à partir de graines. Ce milieu est décrit par GAMBORG D.L., MILLER R et OJINA K. dans Exper. Cell.Res. 50 , 151 - 1968). On met environ 20 graines par boîte.

L'hormone de croissance est extraite de la manière décrite dans l'exemple 1 des cals,des plantes obtenues à partir de ces cals ou des descendants des plantes obtenues à partir de ces cals.

## Exemples 3 et 4

On répète les exemples 1 et 2 mais au lieu du vecteur d'expression pc hGH 800 on emploie le vecteur d'expression "gène hGH sous cloné dans pBR 322" (appelée ci-après pg hGH N) dont la préparation et décrite dans Nucleic acids Res. 9 (1981) 3719 - 3730 par M. DE NOTO, D.D. MOORE et H. GOODMAN.

Ce vecteur pg hGH N est dû à l'insertion du gène de l'hGH au site ECO RI du pBR 322.

La longueur du fragment inseré qui contient le gène est de 2.600 pb.

Le gène contient 5 exons et son promoteur naturel. Il est inséré dans le sens opposé à celui du cDNA dans le vecteur pc hGH 800 des exemples 1 et 2.

La structure du vecteur pg hGH N est représentée à la figure 2 dans laquelle 1,2,3,4 et 5 représentent des exons et a,b,c et d représentent des introns.

L'hôte de ce plasmide est également la souche E. Coli K 12-HB 101.

## Exemples 5 à 8

On brise l'enveloppe de graines de betterave (BETA VULGARIS L. var. Mavigo) et on retire les graines. On traite les graines pendant 2 min par du $Hg_2Cl_2$ à 2 %. Après cinq lavages à l'eau stérile on traite les graines pendant 30 minutes par de l'hypochlorite de sodium (13 % de chlore actif). On lave ensuite les graines stérilisées à l'eau stérile jusqu'à un pH de 6. On les sèche ensuite en atmosphere stérile à température ambiante jusqu'à ce que la teneur en humidité des graines soit inférieure à 5 % en poids.

On prépare ensuite une solution d'ADN, on traite les graines avec cette solution et on extrait l'hormone de croissance de la manière décrite dans les exemples 1 à 4.

## Exemples 9 à 12

On répète les exemples 1 à 4 mais en remplaçant les graines d'orge par des graines de poivron vert (CAPTICUM ANNUUM L., var. fasciculatum) qu'on traite de la manière suivant avant de les faire incuber de la manière décrite dans les exemples 1 à 4 dans la solution d'ADN.

On met les graines pendant 40 minutes dans de l'hypochlorite de sodium (13 % de chlore actif).

On lave les graines à l'eau stérile jusqu'à un pH de 6.

Si la teneur en humidité est inférieure à 5 % en poids un séchage ultérieur n'est pas nécessaire.

## Exemples 13 à 16

On répète les exemples 1 à 4 mais en remplaçant les graines d'orge par des graines de soja (GLYCINE MAX L. var. Japan black).

Avant de les incuber dans la solution d'ADN de la manière décrite dans les exemples 1 à 4 on les met pendant 9 minutes au maximum dans du $Hg_2Cl_2$ 2 % et on les soumet à dix lavages rapides à l'eau stérile.

Le degré d'humidité des graines à incuber est inférieure à 5 % en poids.

## Exemples 17 à 20 .

On répète les exemples 1 à 4 mais en remplaçant les graines d'orge par des graines d'ARABIDOPSIS THALIANA. (mutant V 131 er. pyr.gl.) qu'on traite de la manière suivante.

On place les graines dans des godets en porcelaine et on les recouvre par une solution d'hypochlorite de calcium à 5 % préablablement filtrée sur millipore (0,45 $\mu$m). Après 10 minutes, on enlève la solution d'hypochlorite de calcium et on rince les graines 4 à 5 fois avec de l'eau stérile. On transfère ensuite, en conditions de stérilité, les graines stérilisées, dans des puits d'une plaque en porcelaine placée dans une boîte de Pétri de verre de taille appropriée stérile.

Le degré d'humidité des graines est inférieure à 5 % en poids.

On fait incuber les graines traitées, en lots de plusieurs, de la manière décrite dans les exemples 1 à 4 dans la solution d'ADN mais en ajoutant de la thiamine au milieu de culture "Brown" auquel on a déjà ajouté de l'agar jusqu'à obtenir une concentration finale de milieu de $5.10^{-5}$ M.

### Exemples 21 à 24

On répète les exemples 1 à 4 mais en remplaçant les graines d'orge par des graines de riz (ORYZA SATIVA, var. Japonica, cv Honnen Wase) et en les traitant de la manière suivante avant l'incubation dans la solution d'ADN.

On décortique d'abord soigneusement à la pince les graines sèches. Après l'enlèvement du péricarpe on traite les graines par de l'hypochlorite de sodium (13 % de chlore actif) additionné de Tween 20 (10 $\mu$l/20 ml) pendant 40 minutes après quoi on lave les graines à l'eau stérile jusqu'à un pH de 6.

Le degré d'humidité des graines lavées est inférieure à 5 % en poids. Il s'est avéré qu'une concentration de l'ADN dans la solution de 200 $\mu$g/ml est suffisante.

### Exemples 25 à 28

On agite des graines de fléole (PHLEUM PRATENSE var. erecta, cv RVP) pendant 3 heures dans de l'eau, puis on les pèle sous une loupe binoculaire. On stérilise ensuite les graines par de l'hypochlorite de sodium (13 % de chlore actif). On lave les graines et on les sèche aseptiquement à température ambiante pendant deux heures jusqu'à ce que le degré d'humidité des graines soit inférieure à 5 % en poids.

On traite ensuite les graines de la manière décrite dans les exemples 1 à 4, abstraction faite du traitement des graines avant l'incubation.

## Revendications

1. Procédé de traitement de matériel végétal afin d'obtenir l'expression d'au moins un gène dans des cellules végétales, selon lequel on stérilise d'abord le matériel végétal et on le met ensuite à incuber dans une solution d'ADN possédant au moins un gène étranger à exprimer et/ou le cADN de ce gène, caractérisé en ce qu'on met à incuber au moins une graine sèche de plante supérieure, au moins une spore sèche de plante inférieure ou au moins un embryon de planté excisé sec, dont la teneur en humidité est inférieure à 10 % en poids, au moins jusqu'à ce que l'embryon ait triplé de taille, dans une solution aqueuse d'ADN comprenant le gène étranger complet et/ou le cADN de ce gène.

2. Procédé de traitement selon la revendication 1, caractérisé en ce qu'on met à incuber au moins une graine, au moins une spore ou au moins un embryon, dont la teneur en humidité est inférieure à 5 % en poids.

3. Procédé selon l'une ou l'autre des revendications 1 et 2, caractérisé en ce qu'on sèche le matériel végétal avant de l'incuber dans la solution d'ADN jusqu'à ce que la teneur en humidité du matériel végétal soit inférieure à la teneur maximale susdite.

4. Procédé selon l'une ou l'autre des revendications 1 à 3, caractérisé en ce qu'on fait incuber des graines pelables et on les pèle avant de les incuber dans la solution d'ADN.

5. Procédé selon l'une ou l'autre des revendications 1 à 4, caractérisé en ce qu'on met à incuber dans la solution d'ADN pendant les premières 72 heures du développement de la plantule à partir du germe.

6. Procédé selon l'une ou l'autre des revendications 1 à 5, caractérisé en ce qu'on fait incuber dans une solution saline d'ADN.

7. Procédé selon l'une ou l'autre des revendications 1 à 6, caractérisé en ce qu'on utilise comme solution d'ADN une solution d'ADN comprenant au moins un gène étranger eucaryotique et/ou le cADN de ce gène.

8. Procédé selon la revendication 7, caractérisé en ce qu'on utilise une solution d'ADN comprenant le gène codant pour l'hormone de croissance humaine (hGH) et/ou le cADN ce ce gène.

9. Procédé selon l'une ou l'autre des revendications 1 à 8, caractérisé en ce qu'on utilise une solution d'ADN comprenant le gène étranger et/ou le cADN de ce gène inséré dans un vecteur.

10. Procédé selon l'une ou l'autre des revendications 1 à 9, caractérisé en ce qu'on utilise une solution d'ADN comprenant non seulement le gène étranger et/ou le cADN de ce gène mais également un gène de sélection et/ou le cADN d'un tel gène.

11. Procédé selon les revendications 8 à 10, caractérisé en ce qu'on utilise comme solution d'ADN une solution comprenant le plasmide pc hGH 800 comprenant le gène de l'hGH au site HIND III du pBR 322.

12. Procédé selon les revendications 8 à 10, caractérisé en ce qu'on utilise comme solution d'ADN une solution comprenant le plasmide pg hGH N comprenant le gène de l'hGH inséré au site Eco RI du pBR 322.

13. Procédé selon l'une ou l'autre des revendications 1 à 12, caractérisé en ce qu'on met la graine ou la spore dans une solution d'ADN comprenant au moins 1 μg d'ADN par ml de solution.

14. Procédé selon la revendication 13, caractérisé en ce qu'on met le matériel végétal à incuber dans une solution d'ADN comprenant au moins 400 μg d'ADN par ml.

15. Graine, spore ou embryon de plante excisé dans laquelle ou lequel s'exprime un gène étranger, cette graine ou spore ou cet embryon ayant absorbé selon le procédé selon l'une ou l'autre des revendications 1 à 14 de l'ADN comprenant le gène étranger à exprimer ou le cADN de ce gène.

16. Graine, spore ou embryon de plante selon la revendication 15, caractérisé en ce qu'elle ou qu'il a absorbé l'ADN comprenant le gène complet codant pour l'hormone de croissance humaine ou le cADN de ce gène.

17. Plantes, cals ou cellules obtenus à partir d'une graine, d'une spore ou d'un embryon de plante excisé, traité selon le procédé selon l'une ou l'autre des revendications 1 à 14, et dérivés ou descendants de ces plantes.

## Fig. 1

## Fig. 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,X | NATURE, vol. 249, 3 mai 1974, pages 17-21; L. LEDOUX et al.: "DNA-mediated genetic correction of thiamineless Arabidopsis thaliana" | 1-3,5, 6,13-15,17 | C 12 N  15/00 A 01 H   1/00 |
| Y | Idem<br><br>* Page 17, colonne de droite, ligne 30 - page 18, colonne de gauche; page 20, colonne de gauche, lignes 3-15 * | 4,7-13 ,16 | |
| X | NATURWISSENSCHAFTEN, vol. 59, no. 8, 1972, pages 348-355, Springer Verlag; D. HESS: "Transformationen an höheren Organismen" * Page 349, colonne de droite, lignes 19-34; page 351 - page 352 * | 1-3,7, 15,17 | |
| Y | Idem | 4-6,8-14,16 | |
| X | EP-A-0 174 166 (AGRIGENETICS) * Page 34, lignes 28-31; page 39, lignes 10-12 * | 15,17 | |

DOMAINES TECHNIQUES RECHERCHES (Int. Cl.4)

C 12 N
A 01 H

---

-/-

Le présent rapport de recherche a été établi pour toutes les revendications

| Lieu de la recherche | Date d'achèvement de la recherche | Examinateur |
|---|---|---|
| LA HAYE | 20-07-1987 | MADDOX A.D. |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | NATURE, vol. 218, 29 juin 1968, pages 1256-1259; L. LEDOUX et al.: "Integration and replication of DNA of M. lysodeikticus in DNA of Germinating Barley" <br> * Page 1256, colonne de droite, lignes 1-20 * | 1-6,13 -17 | |
| | --- | | |
| Y | EP-A-0 140 504 (STAUFFER) <br> * Page 4, ligne 27 - page 5, ligne 29 * | 9,10 | |
| | --- | | |
| Y | WO-A-8 301 176 (INTERNATIONAL PLANT RESEARCH INSTITUTE) <br><br> * Page 10, ligne 23 - page 12, ligne 14; page 13, ligne 11 - page 14, ligne 27; revendications 1,20 * | 1-7,9, 10,13- 15,17 | DOMAINES TECHNIQUES RECHERCHES (Int. Cl.4) |
| | --- | | |
| Y | NATURE, vol. 313, 28 février 1985, pages 810-812; J.T. ODELL et al.: "Identification of DNA sequences required for activity of the cauliflower mosaic virus 35S promoter" <br> * Colonne de gauche, alinéa 3 * | 8,9,10 ,16 | |
| | ---     -/- | | |

Le présent rapport de recherche a été établi pour toutes les revendications

| Lieu de la recherche <br> LA HAYE | Date d'achèvement de la recherche <br> 20-07-1987 | Examinateur <br> MADDOX A.D. |
|---|---|---|

**RAPPORT DE RECHERCHE EUROPEENNE**

Office européen
des brevets

Numéro de la demande

EP 87 20 0474

Page 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | SCIENCE, vol. 205, 10 Août 1979, pages 602-607, AAAS; J.A. MARTIAL et al.: "Human growth hormone: complementary DNA cloning and expression in bacteria" * En entier * | 11 | |
| D,Y | CHEMICAL ABSTRACTS, vol. 95, 26 octobre 1981, page 191, résumé no. 145488m, Columbus, Ohio, US; F.M. DENOTO et al.: "Human growth hormone DNA sequence and mRNA structure: possible alternative splicing", & NUCLEIC ACIDS RES. 1981, 9(15), 3719-30 * En entier * | 12 | |
| A | CHEMICAL ABSTRACTS, vol. 86, 17 janvier 1977, page 206, résumé no. 13946p, Columbus, Ohio, US; D. HESS et al.: "Genetic engineering and plant breeding - illusion and reality", & PLANT FOODS HUM. NUTR. 1976 26(1-3), 271-92 * En entier * | 1-6,9, 10,13- 15,17 | DOMAINES TECHNIQUES RECHERCHES (Int. Cl.4) |
| A | WO-A-8 501 856 (DE WET) | | |

--- -/-

Le présent rapport de recherche a été établi pour toutes les revendications

| Lieu de la recherche | Date d'achèvement de la recherche | Examinateur |
|---|---|---|
| LA HAYE | 20-07-1987 | MADDOX A.D. |

## DOCUMENTS CONSIDERES COMME PERTINENTS

Page  4

| Catégorie | Citation du document avec indication, en cas de besoin, des parties pertinentes | Revendication concernée | CLASSEMENT DE LA DEMANDE (Int. Cl.4) |
|---|---|---|---|
| A | INTERNATIONAL REVIEW OF CYTOLOGY, supplément 11B, 1980, pages 47-80, Academic Press, Inc.; C.I. KADO et al.: "Genetic modification of plant cells through uptake of foreign DNA" * Pages 48-53; pages 68-74 * | 1-17 | |
| A | STADLER SYMPOSIUM, vol. 13, 1981, pages 39-51, University of Missouri, Columbia 39, US; M.-D. CHILTON et al.: "Tailoring the agrobacterium Ti plasmid as a vector for plant genetic engineering" | 1-17 | |
| A | EP-A-0 145 338  (AGRIGENETICS) * Page 24,  alinéa 3 - page 25, alinéa 1; page 32, dernière ligne - page 33, ligne 1; revendication 57 * | 1-17 | DOMAINES TECHNIQUES RECHERCHES (Int. Cl.4) |
| A | CHEMICAL ABSTRACTS, vol. 94, 1981, page 451, abrégé no. 99947q, Columbus, Ohio, US; D. HESS: "Attempts to transfer kanamycin resistance of bacterial plasmid origin in Petunia hybrida using pollen as vectors", & BIOCHEM. PHYSIOL. PFLANZ 1981, 176(4), 324-30 * Résumé * | 1-17 | |

Le présent rapport de recherche a été établi pour toutes les revendications

| Lieu de la recherche | Date d'achèvement de la recherche | Examinateur |
|---|---|---|
| LA HAYE | 20-07-1987 | MADDOX A.D. |